# EUROPÄISCHE PATENTSCHRIFT

(11) **EP 3 156 377 B1**
(45) Veröffentlichungstag und Bekanntmachung des Hinweises auf die Patenterteilung: **29.04.2020**
(21) Anmeldenummer: 16192984.9
(22) Anmeldetag: 10.10.2016
(51) Int. Cl.: C22C 5/04, C03B 23/043, C03B 23/045, C03B 23/049, C03B 23/09

(54) **WOLFRAM-HALTIGER FORMDORN ZUR GLASFORMUNG**
WOLFRAM-CONTAINING FORMING MANDREL FOR GLASS FORMING
MANDRIN DE FORMAGE CONTENANT DU TUNGSTÈNE POUR LE FORMAGE DU VERRE

(30) Priorität: 13.10.2015 DE 102015117422
(43) Veröffentlichungstag der Anmeldung: 19.04.2017
(73) Patentinhaber: Schott AG, 55122 Mainz (DE)
(72) Erfinder: Witte, Jörg, 64319 Pfungstadt (DE); Jud, Xaver, 9217 Neukirch a.d. Thur (CH); Humbertjean, Alexander, 9032 Engelburg (CH)
(74) Vertreter: Blumbach · Zinngrebe Patentanwälte PartG mbB

(56) Entgegenhaltungen:
- EP-B1- 1 809 579
- WO-A1-2012/085619
- JP-A- S5 617 933
- JP-A- 2008 280 187
- US-A- 2 376 742
- US-A- 3 371 409
- US-A1- 2003 046 956
- US-A1- 2011 016 925
- US-A1- 2015 114 043

## Beschreibung

Die Erfindung betrifft allgemein die Herstellung von Glasprodukten. Insbesondere betrifft die Erfindung ein Formwerkzeug gemäß Anspruch 1, umfassend einen wolfram-haltigen Formdorn und ein Verfahren gemäß Anspruch 14 zur Heißformgebung von Glas. Die auf diese Weise erhaltenen Glasprodukte können beispielsweise als Pharmapackmittel verwendet werden.

Im Zuge der Herstellung von hohlkörperförmigen Glasprodukten ist die Heißformgebung ein wesentlicher Verfahrensschritt. Der Ablauf der Heißformgebung umfasst dabei üblicherweise mehrere aufeinander folgende Aufwärm- und Formprozesse, mit welchen ausgehend von Rohrglas-Körpern die gewünschte Endgeometrie geschaffen wird.

Hohlkörperförmige Glasprodukte, welche im medizinischen Bereich verwendet werden sollen, unterliegen dabei üblicherweise sehr hohen Anforderungen in Bezug auf mögliche Verunreinigungen, welche aus der Heißformgebung resultieren können. Hieraus folgen sehr hohe Anforderungen an die bei der Heißformgebung verwendeten Formwerkzeuge. Diese hohen Anforderungen beziehen sich insbesondere auf die für die Formwerkzeuge verwendeten Materialien. So wird bei der Herstellung von hohlkörperförmigen Glasprodukten üblicherweise ein Konus geformt, wobei ein Formwerkzeug, umfassend einen Formdorn, zur Anwendung kommt. Dem für den Formdorn verwendeten Material kommt dabei eine zentrale Rolle zu.
Bei der Herstellung von Pharmapackmitteln oder bei Heißformgebung von medizinischen Aufbewahrungsgefäßen für Medikamente aus Glas, beispielsweise Ampullen, Spritzen, Fläschchen oder Karpulen, können metallische Formgebungsmaterialien als Formdornmaterial eingesetzt werden.
Reines Wolfram oder Wolfram-Legierungen weisen eine hohe thermomechanische Festigkeit auf, die besonders bei geringen Materialquerschnitten sehr vorteilhaft sind. Dies ist z.B. der Fall bei Formdornen mit einem Durchmesser von weniger als 0,5 mm bis ca. 1 mm. Von daher sind Formdorne umfassend Wolfram oder Wolfram-Legierungen im Kontaktbereich mit dem umzuformenden Glas von Vorteil.

Während der Herstellung von z.B. Spritzen aus Rohrglas-Körpern, welche üblicherweise auf getakteten Maschinen, beispielsweise auf Rundtaktmaschinen, erfolgt, erwärmt sich der Formdorn bei der Heißformgebung auf Temperaturen von etwa 800°C bis 900°C. Aufgrund der geringen Taktzeiten ist das Material dabei einem häufigen und raschen Temperaturwechsel unterzogen. Der Einfluss von Temperatur, Glasverdampfungsprodukten, Luft, Feuchtigkeit und die tribologische Belastung durch das angepresste, teigige Glas während der Heißformgebung können daher einen Materialabtrag am Formdorn bewirken.

Dieser Materialabtrag des Formdorns, insbesondere im Kontaktbereich mit dem umzuformenden Glas-Vorprodukt, ist im Allgemeinen unerwünscht und führt zu einer Veränderung der Außengeometrie oder einer Reduktion des Durchmessers des Formdorns, wobei die Abnahme des Durchmessers zudem häufig nicht gleichmäßig axial ausgeprägt ist. Dieser Materialabtrag hat verschiedene weitere negative Auswirkungen.

So führt der Materialabtrag dazu, dass die für die Formgebung vorgegebenen Sollmaße durch die Veränderung der Außengeometrie des Formdorns im Zeitverlauf immer weniger eingehalten und die geforderten Kanaldimensionen damit nur für einen begrenzten Zeitraum eingehalten werden können. Die Standzeit des Formdorns ist damit vergleichsweise gering und/oder zusätzlicher Aufwand für nachträgliche Justagen wird erforderlich. Ferner kann das abgetragene Material in das Innere des gerade geformten Glasproduktes gelangen. So kann der Materialabtrag bei der Konusformung von Spritzen beispielsweise in den sogenannten Konuskanal gelangen.

Pharmakologische Wirkstoffe, die in den Spritzen gelagert werden, können allerdings mit den wolframhaltigen Verunreinigungen im Innenraum des Glasproduktes in Wechselwirkung treten, wodurch deren Wirksamkeit verändert werden kann. Für bestimmte pharmazeutische Wirkstoffe werden daher zunehmend Glasgefäße gefordert, die einen bestimmten Wolframgehalt unterschreiten oder sogar gänzlich frei von Wolfram sind.

Vor diesem Hintergrund sind Formdorne entwickelt worden, welche frei von Wolfram sind. So schlägt zum Beispiel das Dokument EP 1 809 579 B1 zur Vermeidung von Wolfram-haltigen Verunreinigungen vor, einen Formdorn zu verwenden, der kein Wolfram enthält.

Der Formdorn der US2376742 A beschreibt Platin als ein erstes Legierungselement und Rhodium oder Iridium als weiteres Legierungselement, aber kein Wolfram.

US3371409 A enthält einen Dorn, der eine mit Gold angereicherte Oberfläche aufweist, die aus einer Platin-Gold-Legierung besteht.

In dem Dokument EP 1 471 040 A1 wird ein Formdorn beschrieben, welcher frei von Wolfram ist und als wesentliches Material Graphit umfasst. Ferner kann der Formdorn andere Materialien wie Silizium-Carbid oder Zirkon-Carbid umfassen und ist damit frei von Wolfram.
Weiterhin wird in dem Dokument WO 2012/039705 A2 ein Formdorn vorgeschlagen, welcher eine Schutzschicht als Auflageschicht umfasst. Das Basismaterial kann dabei wolframhaltig sein. Als Schichtmaterial werden ein Neutralglas-eigenes Material bzw. ein Glas vorgeschlagen, welches ein Bestandteil des umzuformenden Glases ist, um auf diese Weise Fremdverunreinigungen auszuschließen.
Im Allgemeinen weisen derartige Beschichtungen einen gewissen Verschleiß auf, so dass der nachteilige Effekt des Materialabtrags des Formdorns kaum verhindert werden kann. Dadurch ist die Standzeit eines derartigen Formdorns eher gering. Weiterhin kann aufgrund der hohen mechanischen Belastung eine Rissbildung des Schichtmaterials oder auch ein Abplatzen von Schichtbestandteilen auftreten, so dass die Standzeit des Formdorns weiter verringert wird.

Andererseits stellt für eine Vielzahl von geformten Glasprodukten ein geringer Wolfram-Anteil im Innenbereich des umgeformten Teiles keine allzu großen Nachteile dar, sofern der Wolfram-Anteil in einem vorbestimmten Bereich bleibt oder einen tolerierbaren Grenzwert nicht überschreitet.

Hieraus ergibt sich die Aufgabe der vorliegenden Erfindung, die darin zu sehen ist, ein Material für ein Formwerkzeug, insbesondere für einen Formdorn, sowie ein Verfahren und eine Vorrichtung zur Formung hohlkörperförmiger Glasprodukte zur Verfügung zu stellen.
Das Eintragen von unerwünschten Verunreinigungen, insbesondere von Wolfram, in das umzuformende Glasprodukt durch das Formwerkzeug, insbesondere durch den Formdorn, soll dabei in einem vorbestimmten Rahmen bleiben. Insbesondere soll der Innenbereich bzw. der Innenkonus des umgeformten Glasproduktes einen vorgegebenen Grenzwert von Verunreinigungen durch das Formwerkzeug, insbesondere durch den Formdorn, nicht überschreiten.

Ferner soll das Formwerkzeug, insbesondere der Formdorn, eine hohe Standzeit aufweisen. Dabei soll der Materialabtrag möglichst verhindert werden, so dass eine besonders hohe Standzeit bei gleichbleibender Qualität ohne zusätzlichen Justageaufwand erreicht werden kann.

Weiterhin soll auch das Abplatzen von äußeren Bestandteilen des Formwerkzeuges, insbesondere des Formdorns, weitgehend ausgeschlossen werden.

Schließlich soll auch die Gefahr von Rissbildungen auf der Oberfläche des Formdorns vermieden werden.

Überraschend einfach wird diese Aufgabe durch den Gegenstand der unabhängigen Ansprüche gelöst. Bevorzugte Ausführungsformen und Weiterbildungen der Erfindung sind den jeweiligen Unteransprüchen zu entnehmen.

Demgemäß betrifft die Erfindung ein Formwerkzeug zum Umformen von hohlkörperförmigen Glas-Vorprodukten, umfassend einen Formdorn zum Umformen wenigstens eines Abschnittes eines erhitzten Bereiches des Glas-Vorproduktes.

Dabei ist der Formdorn aus einer Legierung gebildet, welche zumindest ein erstes temperaturstabiles Kernmaterial aufweist. Vorzugsweise handelt es sich um einen massiven Formdorn, um eine größtmögliche Stabilität zu erreichen. Unter dem Begriff Kernmaterial wird im Zusammenhang mit dieser Schrift das Grundmaterial bzw. Basismaterial des Formdorns verstanden, aus dem dieser gefertigt wird.

Als Kernmaterial des Formdorns ist besonders ein Material geeignet, welches eine hohe Warmfestigkeit und ein hohes Elastizitätsmodul aufweist, so dass es für die mechanischen Belastungen auch bei Temperaturen im Bereich von 400°C oder mehr geeignet ist. Ferner soll es beständig sein gegenüber Säuren und Glasverdampfungsprodukten wie beispielsweise Borate und eine Oxidationsbeständigkeit an Luft bis 400°C aufweisen, um den Materialabtrag während oder durch die Heißformgebungsprozesse zum Umformen von hohlkörperförmigen Glas-Vorprodukten zu vermeiden.

Das Kernmaterial ist daher ausgewählt aus der Gruppe der Edelmetalle, insbesondere der Platingruppenelemente. Das Kernmaterial kann auch eine Legierung umfassen. Ein geeignetes Kernmaterial stellen die Edelmetalle oder deren Legierungen dar, beispielsweise Platin.

Neben dem Kernmaterial weist der Formdorn ein weiteres Legierungselement auf. Dieses ist ausgewählt aus der Gruppe umfassend Wolfram, Zirkonium, Rhodium, Molybdän oder Rhenium.

Die Erfinder haben herausgefunden, dass neben den bekannten Formdornen aus Wolfram auch Formdorne, welche Legierungen aus Platingruppenelementen umfassen, sehr gut als Formwerkzeug, insbesondere als Kernmaterial für einen Formdorn, geeignet sind, da sie eine hohe mechanische Festigkeit aufweisen.

So konnte nachgewiesen werden, dass schon geringe Zusätze des weiteren Legierungselements Wolfram, Zirkonium, Rhodium, Molybdän oder Rhenium das mechanische und strukturelle Verhalten des Formdorns signifikant verbessern können. Besonders vorteilhaft zeigt sich die mechanische und strukturelle Verbesserung des Formdorns dann, wenn als Kernmaterial das Material Iridium ausgewählt wird.

Völlig unerwartet war bei einem derartigen Formwerkzeug, welches als Kernmaterial Iridium umfasste und als weiteres Legierungselement Wolfram, dass der Eintrag von Wolfram in das umzuformende Glasprodukt sehr niedrig ist. Dies ist vor allem deshalb überraschend, da allgemein bekannt ist, dass die Verwendung von wolframhaltigen Materialien als Formwerkzeug, insbesondere als Formdorn, zu stärkeren Wolframverunreinigungen des umgeformten Produktes, insbesondere des Innenbereiches des umgeformten Produktes, führt. Vor diesem Hintergrund empfiehlt etwa die Schrift EP 1 809 579 B1 die Verwendung von wolframfreien Formdornen.

Der Innenbereich, insbesondere der Innenkonus des umgeformten Glasproduktes, kann in überraschender Weise daher nahezu frei von Verunreinigungen, insbesondere frei von Verunreinigungen mit Wolfram, gehalten werden, obwohl der Formdorn Wolfram-Anteile aufweist.

Das Kernmaterial des Formdorns kann neben Iridium auch Palladium, Platin, Rhodium, Rhenium und/oder Ruthenium oder eine Legierung aufweisend Iridium, Palladium, Platin, Rhodium, Rhenium und/oder Ruthenium umfassen.

In einer Ausführungsform umfasst das Kernmaterial wenigstens 20 Gew.-% Platin oder eine Legierung mit wenigstens 20 Gew.-% Platin.

In einer weiteren Ausführungsform umfasst das Kernmaterial wenigstens 5 Gew.-% Rhodium oder eine Legierung mit wenigstens 5 Gew.-% Rhodium.

In einer weiteren, bevorzugten Ausführungsform umfasst das Kernmaterial eine Platin-Rhodium-Legierung mit wenigstens 20 Gew.-% Platin, bevorzugt 25 Gew.-% Platin und besonders bevorzugt 30 Gew.-% Platin und wenigstens 5 Gew.-% Rhodium, bevorzugt wenigstens 7 Gew.-% Rhodium und besonders bevorzugt wenigstens 10 Gew.-% Rhodium.

In einer nochmaligen weiteren Ausführungsform umfasst das Kernmaterial eine Platin-Nickel-Legierung mit wenigstens 5 Gew.-% Nickel.

In einer nochmaligen weiteren, bevorzugten Ausführungsform umfasst das Kernmaterial eine Platin-Iridium-Legierung mit wenigstens 5 Gew.-% Iridium, bevorzugt wenigstens 10 Gew.-% Iridium und besonders bevorzugt wenigstens 20 Gew.-% Iridium.

In einer nochmaligen weiteren, bevorzugten Ausführungsform umfasst das Kernmaterial wenigstens 20 Gew.-% Iridium, bevorzugt wenigstens 50 Gew.-% Iridium, besonders bevorzugt wenigstens 70 Gew.-% Iridium und ganz besonders bevorzugt wenigstens 90 Gew.-% Iridium als Legierungselement.

Die Erfinder haben herausgefunden, dass bereits sehr geringe Zusätze von Legierungselementen wie Wolfram, Zirkonium, Rhodium, Molybdän oder Rhenium das mechanische und strukturelle Verhalten der Legierung für den Formdorn deutlich verbessern kann. Der Umfang dieser Legierungselemente liegt dabei vorteilhaft in einem Bereich von wenigstens 0,1 Gew.-%, bevorzugt von wenigstens 0,3 Gew.-%, besonders bevorzugt 0,5 Gew.-% und ganz besonders bevorzugt wenigstens 0,9 Gew.-% sowie höchstens 10 Gew.-%, bevorzugt höchstens 6 Gew.-% und besonders bevorzugt höchstens 5 Gew.-%.

In einer ganz besonders bevorzugten Ausführungsform ist als Kernmaterial Iridium ausgewählt und als Legierungselement Wolfram.

Iridium oder Hoch-Iridium-haltige Legierungen weisen eine sehr hohe Härte auf, die zum einen durch das Material als solches gegeben ist, aber auch durch Kaltverfestigung bei der Herstellung erzielt werden kann. Aufgrund der hohen Rekristallisiationstemperatur von Iridum bleibt diese Festigkeit bzw. Härte auch während des Einsatzes bei Temperaturen um 800°C oder 900°C, wie sie bei der Heißformgebung auftreten können, weitgehend erhalten.

Wolfram zeichnet sich durch eine hohe Beständigkeit gegenüber Säuren wie beispielsweise HF und HCl aus und wird nur gering durch Schwefelsäure angegriffen. Weiterhin weist es eine gute Oxidationsbeständigkeit an Luft bis 400°C auf. Zudem sind eine hohe Warmfestigkeit und ein hohes Elastizitätsmodul günstig für eine Verwendung als Legierungsmaterial für einen Formdorn.

Es hat sich gezeigt, dass bereits sehr geringe Anteile von Wolfram als Legierungselement positiven Einfluss auf die Festigkeit des Dorns aufweisen. Die sehr geringen Anteile von Wolfram liegen dabei bei einem Gewichtsanteil ab etwa 0,01 Gew.-%.

Sehr vorteilhaft ist ein Material für den Formdorn daher, welches eine Iridium-Wolfram-Legierung mit wenigstens 0,01 Gew.-%, 0,05 Gew.-%, 0,1 Gew.-%, bevorzugt wenigstens 0,3 Gew.-%, besonders bevorzugt wenigstens 0,5 Gew.-% und ganz besonders bevorzugt wenigstens 1 Gew.-% Wolfram umfasst. Dabei ist es weiterhin vorteilhaft, wenn der Wolfram-Anteil höchstens 10 Gew.-%, bevorzugt höchstens 6 Gew.-% und besonders bevorzugt höchstens 5 Gew.-% beträgt. Das sehr geeignete Material für den Formdorn kann beispielsweise eine Iridium-Wolfram-Legierung mit 0,96 Gew.-%, 1,92 Gew.-% oder 3,84 Gew.-% Wolfram umfassen.

Ein derartiges Material weist eine erhöhte Zugfestigkeit und eine reduzierte Bruchdehnung auf. So weist eine Iridium-Wolfram-Legierung mit 0,96 Gew.-% Wolfram etwa eine Dehngrenze von 16,271 kN/cm² auf. Die Zugfestigkeit beträgt 39,989 kN/cm² und die Bruchdehnung 16,823 kN/cm².

Weiterhin konnte beobachtet werden, dass sich die Korngröße einer derartigen Iridium-Wolfram-Legierung reduzierte und sich die Rekristallisationstemperatur erhöhte. Dieses wirkte sich sehr positiv auf das Abplatzen von äußeren Bestandteilen des Formwerkzeuges und die Gefahr von Rissbildungen auf der Oberfläche des Formdorns aus. Hierdurch konnten die Standzeiten eines Formdorns mit einer Iridium-Wolfram-Legierung als Formdornmaterial deutlich verbessert werden.

Im Vergleich zu den Edelmetallen Platin und Rhodium weist Iridium die höchste Flüchtigkeit in Sauerstoff enthaltener Atmosphäre bei erhöhter Temperatur auf. Die Bildung der Iridiumoxide IrO₂ und IrO₃ erfolgt bei Temperaturen oberhalb von ca. 900°C. Diese Oxide sind flüchtig, sie sublimieren oberhalb von etwa 1.000°C.

Ein Wolfram-Anteil einer Iridium-Wolfram-Legierung zwischen 1 Gew.-% und 3 Gew.-% kann im Vergleich zu reinem Iridium den zeitlichen Massenverlust deutlich reduzieren. Überraschend führt daher eine derartige Iridium-Wolfram-Legierung zum einen zu einer sehr hohen Standzeit, zum anderen aber auch aufgrund des sehr geringen zeitlichen Massenverlustes zu einem sehr geringen Eintrag von Wolfram in den Innenbereich oder den Konuskanal des umzuformenden Glas-Vorproduktes.

Zudem konnte auch die Oxidationsbeständigkeit an Luft deutlich verbessert werden, was einen sehr positiven Einfluß auf den Korrosionsverschleiß des Formdorns im Einsatz hat.

Aus den vorstehend genannten Gründen eignen sich daher Iridium-Legierungen mit einem Legierungszusatz von Wolfram zur Verwendung als Formdorn bei der Heißformgebung des Konuskanals in ganz besonderer Weise.

Derartige Iridium-Wolfram Legierungen können pyrometallurgisch gefertigt werden. Der Formgebungsprozess des Dorns kann mittels Drahtziehen erfolgen, wobei ein Schleifprozess zur Verbesserung der Oberfläche nachgeschaltet sein kann.

Des weiteren sieht die Erfindung eine Vorrichtung zum Umformen von Glasprodukten vor, umfassend
- eine Einrichtung zur lokalen Erhitzung eines Bereichs eines Glas-Vorprodukts bis über dessen Erweichungspunkt, und
- zumindest ein Formwerkzeug zum Umformen wenigstens eines Abschnittes eines mit der Einrichtung zur lokalen Erhitzung erhitzten Bereichs des Glas-Vorprodukts, wobei das Formwerkzeug einen erfindungsgemäßen Formdorn umfasst.

Der Formdorn ist aus einer Legierung gebildet, welche zumindest ein erstes temperaturstabiles Kernmaterial und zumindest ein weiteres Legierungselement aufweist, wobei das Kernmaterial wenigstens 50 Gew.-% umfasst und ausgewählt ist aus der Gruppe der Edelmetalle, insbesondere der Platingruppenelemente, und wobei das weitere Legierungselement ausgewählt ist aus der Gruppe umfassend Wolfram, Zirkonium, Rhodium, Molybdän und/oder Ruthenium oder Rhenium.

Während des Umformprozesses kann ein Bereich der Oberfläche des Formdorns im direkten Kontakt mit dem Glas-Vorprodukt stehen.

Die Vorrichtung kann einen Brenner zur lokalen Erhitzung umfassen, wobei ferner eine Rotationseinrichtung vorgesehen ist, um das Formwerkzeug und das Glas-Vorprodukt relativ zueinander zu rotieren.

Das Formwerkzeug kann weiterhin ein Walzenpaar umfassen, welches so angeordnet ist, dass die Walzen des Walzenpaares auf der Oberfläche eines mittels der Rotationseinrichtung in

Rotation versetzten Glas-Vorproduktes abrollen, wobei von dem Laserlicht ein zwischen den Walzen liegender Bereich auf dem Umfang des Glas-Vorproduktes beleuchtet wird.

Die Vorrichtung kann auch in einer weiteren Ausführungsform einen Laser zur lokalen Erhitzung umfassen. Hierbei ist das Formwerkzeug bevorzugt so ausgebildet, dass ein Oberflächenbereich des umzuformenden Abschnittes des Glas-Vorproduktes nicht durch das Formwerkzeug abgedeckt wird, wobei der Laser oder eine dem Laser nachgeschaltete Optik so angeordnet ist, dass das Laserlicht bei der Umformung auf den nicht vom Formwerkzeug abgedeckten Bereich eingestrahlt wird, und wobei eine Steuereinrichtung vorgesehen ist, welche den Laser ansteuert, so dass zumindest zeitweise das Glas-Vorprodukt während der Umformung durch das Laserlicht erwärmt wird

Damit eine Erwärmung des Glases eines in der Vorrichtung umzuformenden Glas-Vorprodukts erfolgt, wird ein Laser verwendet, welcher Licht einer Wellenlänge emittiert, für welche das Glas des Glas-Vorprodukts höchstens teiltransparent ist, so dass das Licht zumindest teilweise im Glas absorbiert wird.

Das mit einer derartigen Vorrichtung durchführbare Verfahren zum Umformen von Glasprodukten basiert entsprechend darauf, dass
- lokal ein hohlkörperförmiges Glas-Vorprodukt bis über dessen Erweichungspunkt erhitzt wird, und
- mit zumindest einem Formwerkzeug wenigstens ein Abschnitt eines mit einer Einrichtung zur lokalen Erhitzung erhitzten Bereiches des Glas-Vorproduktes umgeformt wird,
- wobei das Formwerkzeug einen erfindungsgemäßen Formdorn wie vorstehend beschrieben umfasst, und
- wobei das Glas-Vorprodukt und das Formwerkzeug relativ zueinander mittels einer Rotationsvorrichtung rotiert werden.

Das erfindungsgemäße Formwerkzeug ist besonders geeignet für die kontaminationsarme Herstellung von Pharmapackmitteln aus Glas.

Demgemäß umfasst die Erfindung auch eine Spritze aus Glas, welche hergestellt oder herstellbar ist mit einem Verfahren wie vorstehend beschrieben, und welche, insbesondere im Innenbereich oder im Bereich des umgeformten Innenkonus, nur sehr geringe Verunreinigungen, insbesondere nur sehr geringe Wolfram-Verunreinigungen, aufweist.

Eine mittels des vorstehend genannten Verfahrens oder mittels des vorstehend genannten Formwerkzeuges erzeugte Spritze aus Glas kann innerhalb des Hohlraums daher einen geringen Gehalt an Wolfram aufweisen.

Der Gehalt an Wolfram lässt sich mit der Neutronenaktivierungsanalyse (NAA) ermitteln. Hierbei handelt es sich um eine kernphysikalische Methode zur quantitativen Analyse der Element- oder Isotopenzusammensetzung von Proben, welche hierzu mit Neutronen bestrahlt werden. In der Folge treten die zu bestimmenden Atomkerne der Probe, auch als Analytkerne bezeichnet, mit den Neutronen in eine Wechselwirkung und es können, in Abhängigkeit von der Art der Kernreaktion, verschiedene Produkte entstehen.
Dieser Vorgang kann in einem Forschungsreaktor oder mit Hilfe einer anderen Neutronenquelle durchgeführt werden. Die entstandenen Aktivierungsprodukte können radioaktiv sein und zerfallen dann mit ihrer charakteristischen Halbwertszeit. Sowohl bei Aktivierung als auch beim Zerfall wird Strahlung mit ebenfalls charakteristischen Energien frei, die zur Analyse benutzt werden kann.

Erfindungsgemäß hergestellte Spritzen weisen einen sehr niedrigen Wolfram-Gehalt auf. Bei einer Nachweisgrenze von 3 ng, welche mit herkömmlichen chemischen Analyseverfahren erreichbar ist, lassen sich keine Wolframanteile nachweisen. Bei der Neutronenaktivierungsanalyse liegt die Nachweisgrenze deutlich niedriger, etwa bei 0,03 ng bis hin zu 0,006 ng. In einem Fall konnte ein Wolframanteil von etwa 0,006 ng pro Spritze ermittelt werden.

Daher liegt der Gehalt an Wolfram der Spritze bei > 0, was auf einen, wenn auch sehr geringen, Verschleiß des Formdorns zurückzuführen ist und welcher nach einem Zyklus von 100.000 Umformungen bestimmt wurde. Anhand des Verschleißes errechnet sich eine Verunreinigung einer Spritze durch einen erfindungsgemäßen Umformvorgang, welche in einem Bereich von 0,01 bis 0,03 ng / Spritze liegt.

Der Wolframgehalt einer Spritze liegt daher bei > 0 ng, > 0,006 ng, > 0,01 ng, > 0,03 ng.

Der Gehalt an Wolfram innerhalb des Hohlraums liegt dabei bei höchstens 100 ng, höchstens 50 ng, höchstens 10 ng, bevorzugt höchstens 1 ng und besonders bevorzugt höchstens 0,1 ng pro Spritze.

Eine derartig niedrige Verunreinigung ist für einen Großteil von Glasprodukten unschädlich, so dass der erfindungsgemäße Formdorn aufgrund seiner sehr hohen Standzeiten hier zu deutlichen Kosteneinsparpotentialen beitragen kann.

Der Nachweis der Wolfram-Kontamination erfolgt mittels einer wässrigen Extraktions-Methode, wobei das innere Volumen der Spritze mit gereinigtem Wasser ausgewaschen wird. Das Füllvolumen entspricht dabei dem vorgesehenen Füllvolumen der Spritze. Dabei wird bei erhöhter Temperatur (typisch sind 50°C bis über 100°C) und über eine Dauer von 4 Tagen gearbeitet. Der chemische Nachweis vom Wolfram erfolgt im Eluat mittels ICP-MS.

Ein weiterer Vorteil der Erfindung ist darin zu sehen, dass auf eine Beschichtung des Formdorns im Kontaktbereich verzichtet werden kann und damit ein Unterschied im Ausdehnungsverhalten zwischen dem Kernmaterial und der Beschichtung nicht zu einer geringen Verzahnung führen, wodruch die Gefahr eines Abplatzens von äußeren Bestandteilen des Formdorns weitgehend verringert werden kann. Gleichzeitig ist auch die Gefahr von Rissbildungen minimiert.

Die hohe Standzeit des Formdorns ermöglicht eine Produktion mit gleichbleibend hoher Qualität des umgeformten Glasproduktes, ohne dass häufige, zusätzliche Justagetätigkeiten an der Vorrichtung zum Umformen notwendig werden. So ist eine besonders hohe Qualität der Maßeinhaltung gegeben, da der Materialabtrag des Fordorns reduziert ist. Dadurch kann ein enges Toleranzfeld bezüglich der Geometrie des Glasproduktes eingehalten werden.

Ein weiterer großer Vorteil liegt darin, dass durch den geringeren Korrosionsverschleiß deutlich weniger Partikel im Kontaktbereich mit dem umzuformenden Glas-Vorprodukt, beispielsweise im Kontaktbereich mit dem Konuskanal einer Spritze, abgelagert werden. Gleichzeitig werden deutlich weniger flüchtige Wolframkomponenten in das Innere des hohlkörperförmigen Glasproduktes eingebracht, etwa in den Innenraum der Spritze. Der chemisch analytisch nachweisbare Wolfram-Gehalt wird somit erheblich reduziert. Hierdurch ist das erfindungsgemäße Formwerkzeug, insbesondere der erfindungsgemäße Formdorn, besonders geeignet zur Verwendung in der Heißformgebung von Glasprodukten, welche als Pharmapackmittel eingesetzt werden.

Die Erfindung wird nachfolgend anhand bevorzugter Ausführungsformen und unter Bezugnahme auf die beigefügten Figuren detaillierter beschrieben. Weitere Einzelheiten der Erfindung ergeben sich aus der Beschreibung der dargestellten Ausführungsbeispiele und den angefügten Ansprüchen.

Die Zeichnungen zeigen:
- Fig. 1: Teile einer Vorrichtung zur Umformung von Rohrglas,
- Fig. 2: ein Transmissionsspektrum eines Glases eines Glas-Vorprodukts,
- Fig. 3A-3F: Schnittansichten durch ein Rohrglas im Verlauf des Umformprozesses,
- Fig. 4: einen Abschnitt eines Formdorns in einer Draufsicht, und
- Fig. 5: eine Übersicht über den Massenverlust verschiedener Iridium-Wolfram-Legierungen bei 1.000 °C.

### Detaillierte Beschreibung bevorzugter Ausführungsformen

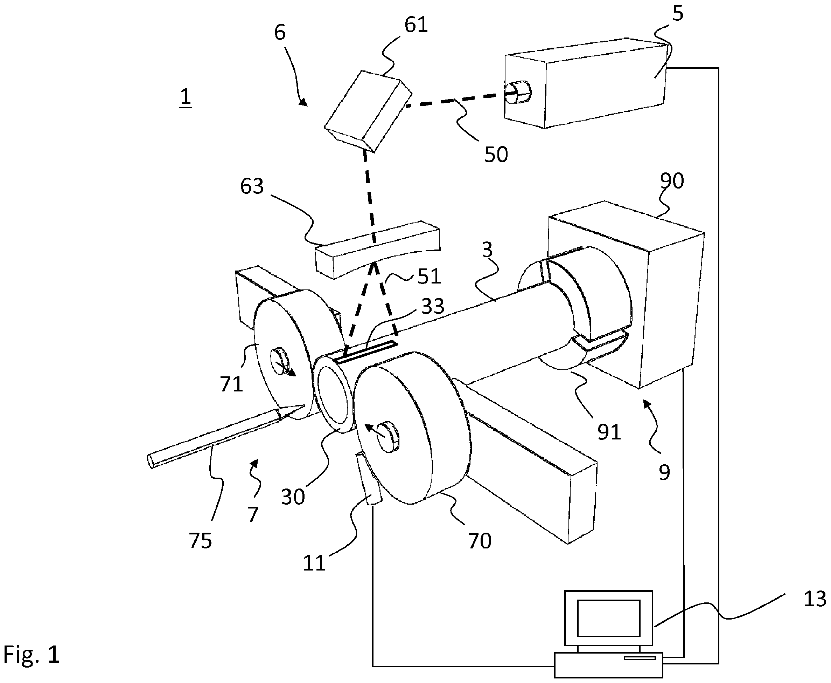

Bei der nachfolgenden detaillierten Beschreibung bevorzugter Ausführungsformen bezeichnen um der Klarheit willen gleiche Bezugszeichen im Wesentlichen gleiche Teile in oder an diesen Ausführungsformen. Zur besseren Verdeutlichung der Erfindung sind die in den Figuren dargestellten bevorzugten Ausführungsformen jedoch nicht immer maßstabsgerecht gezeichnet. In Fig. 1 ist ein Ausführungsbeispiel einer Vorrichtung 1 zur Durchführung des erfindungsgemäßen Verfahrens dargestellt.

Die als Ganzes mit dem Bezugszeichen 1 bezeichnete Vorrichtung des in Fig. 1 gezeigten Ausführungsbeispiels ist zur Umformung von Glas-Vorprodukten in Gestalt von Rohrgläsern 3 ausgebildet. Im Speziellen wird die Vorrichtung zur Herstellung von Glas-Spritzenkörpern verwendet, wobei mit den in Fig. 1 gezeigten Elementen der Vorrichtung 1 aus dem Rohrglas der Konus des Spritzenkörpers geformt wird.

Das Herstellen des Konus aus dem Rohrglas mittels der Vorrichtung 1 basiert darauf, dass lokal ein Bereich eines Rohrglases 3, hier dessen Ende 30, bis über dessen Erweichungspunkt erhitzt und mit zumindest einem Formwerkzeug wenigstens ein Abschnitt des erhitzten Endes umgeformt wird. Im abgebildeten Beispiel umfasst die Einrichtung zur lokalen Erhitzung einen Laser 5, welcher Licht einer Wellenlänge emittiert, für welche das Glas des Rohrglases 3 höchstens teiltransparent ist, so dass das Licht zumindest teilweise im Glas absorbiert wird.

Anstelle des in Fig. 1 gezeigten Lasers 5 zur lokalen Erhitzung eines hohlkörperförmigen Glas-Vorproduktes ist es selbstverständlich auch möglich, andere Einrichtungen zur lokalen Erhitzung zu verwenden, welche im Rahmen der Heißformgebung von Glas typischerweise eingesetzt werden. Dies können beispielsweise auch Brenner, etwa Gasbrenner, sein. Da die Verwendung derartiger Einrichtungen weitgehend bekannt ist, wird auf eine detaillierte Beschreibung an dieser Stelle verzichtet.

Der Laserstrahl 50 nach Fig. 1 wird mittels einer Optik 6 auf das Rohrglas 3 gerichtet. Während des Umform-Prozesses werden das Formwerkzeug 7 und das Glas-Vorprodukt 3 relativ zueinander mittels einer Rotationseinrichtung 9 rotiert. Im Allgemeinen ist es dabei zweckmäßig, wie auch im gezeigten Beispiel das Rohrglas 3 mit Drehachse entlang der axialen Richtung des Rohrglases 3 zu rotieren. Dazu umfasst die Rotationseinrichtung 9 einen Antrieb 90 mit Futter 91, mit welchem das Rohrglas 3 gehaltert wird. Denkbar wäre auch eine umgekehrte Konfiguration, bei welchem das Rohrglas festgehalten wird und das Formwerkzeug 7 um das Rohrglas rotiert. Das Formwerkzeug 7 umfasst bei dem in Fig. 1 gezeigten Ausführungsbeispiel zwei Walzen 70, 71, welche bei Rotation des Rohrglases 3 auf dessen Oberfläche abrollen. Dabei wird das Ende 30 des Rohrglases 30 komprimiert, indem die Walzen in radialer Richtung des Rohrglases 3 aufeinander zu geführt werden. Die radiale Bewegung ist in Fig. 1 anhand von Pfeilen an den Drehachsen der Walzen 70, 71 verdeutlicht.

Weiterhin ist ein Formdorn 75 als Bestandteil des Formwerkzeugs 7 vorgesehen. Dieser Formdorn 75 wird in die Öffnung des Rohrglases 3 an dessen umzuformenden Ende 30 eingeführt. Mittels des Formdorns 75 wird der Konuskanal des Spritzenkörpers geformt. Der Formdorns 75 kann drehbar gelagert sein, um zusammen mit dem Rohrglas 3 zu rotieren. Ebenso möglich ist es, das rotierende Glas über den festgehaltenen Formdorn gleiten zu lassen.

Um ein Anhaften zu vermeiden, kann hierzu, wie generell bei über die Glasoberfläche gleitenden Formwerkzeugen, ein Trenn- oder Schmiermittel verwendet werden, welches die Reibung bei der Gleitbewegung herabsetzt. Weiterhin ist es möglich, ein Schmiermittel zu verwenden, welches bei den bei der Umformung eingesetzten Temperaturen verdampft. Bei der Verwendung eines solchen Schmiermittels können so vorteilhaft Schmier-, beziehungsweise Trennmittelrückstände auf dem fertig gestellten Glasprodukt vermieden werden.

Zwischen den Walzen 70, 71 ist es möglich, den Laserstrahl 50 auf das Rohrglas zu richten, ohne dass der Laserstrahl 50 durch das Formwerkzeug unterbrochen wird. Demgemäß ist das Formwerkzeug so ausgebildet, dass ein Oberflächenbereich des umzuformenden Abschnittes des Rohrglases nicht durch das Formwerkzeug abgedeckt wird, so dass mittels der dem Laser nachgeschalteten Optik 6 das Laserlicht bei der Umformung auf den nicht vom Formwerkzeug abgedeckten Bereich eingestrahlt wird. Im Speziellen wird vom Laserlicht ein zwischen den Walzen 70, 71 liegender Bereich 33 auf dem Umfang des Rohrglases 3 beleuchtet.

Eine Steuereinrichtung 13 steuert den Umformvorgang. Insbesondere wird mittels der Steuereinrichtung 13 der Laser 5 so angesteuert wird, dass zumindest zeitweise das Rohrglas 3 während der Umformung durch das Laserlicht erwärmt wird.

Die Optik 6 der in Fig. 1 gezeigten Vorrichtung 1 umfasst einen Umlenkspiegel 61, sowie eine Zylinderlinse 63.

Mittels der Zylinderlinse 63 wird der Laserstrahl 50 entlang der axialen Richtung des Rohrglases 3 zu einem Fächerstrahl 51 aufgeweitet, so dass der vom Laserlicht ausgeleuchtete Bereich 33 in axialer Richtung des Rohrglases 3 entsprechend gedehnt wird. Da das Rohrglas 3 rotiert, während das Laserlicht eingestrahlt wird, verteilt sich die eingestrahlte Leistung in Umfangsrichtung auf dem Rohrglas, so dass ein zylinderförmiger Abschnitt, beziehungsweise unabhängig von der Form des Glas-Vorprodukts allgemein ein Abschnitt in axialer Richtung entlang der Drehachse erwärmt wird. Dieser Abschnitt hat eine Länge, die vorzugsweise mindestens so groß ist, wie der umzuformende Abschnitt. Letzterer hat eine Länge, die im Wesentlichen durch die Breite der Walzen bestimmt wird. Um spezielle Verteilungen der Laserleistung in axialer Richtung des Rohrglases zu erzielen, kann alternativ oder zusätzlich zur Zylinderlinse 63 auch vorteilhaft ein diffraktives optisches Element verwendet werden.

Der Formungsprozess wird mittels der Steuereinrichtung 13 gesteuert. Unter anderem steuert die Steuereinrichtung 13 die Laserleistung. Weiterhin wird auch die Bewegung der Formwerkzeuge 70, 71, 75 kontrolliert. Ebenfalls gesteuert werden kann auch die Rotationseinrichtung 9, dabei insbesondere die Drehzahl des Antriebs 90, gegebenenfalls auch das Öffnen und Schließen des Futters 91.

Bei der Formung von Spritzenkörpern aus Glas sind für den Laser 5 im allgemeinen Strahlungsleistungen von weniger als 1 Kilowatt ausreichend, um eine schnelle Erwärmung auf die Erweichungstemperatur zu gewährleisten. Nach Erreichen der für die Heißumformung vorgesehenen Temperatur kann dann von der Steuereinrichtung 1 die Laserleistung reduziert werden, so dass die eingestrahlte Laserleistung nur noch die Abkühlung kompensiert. Bei der Herstellung von Spritzenkörpern reichen dazu im Allgemeinen Leistungen zwischen 30 und 100 Watt.

Die Regelung der Laserleistung kann insbesondere auch anhand der Temperatur des Rohrglases 3 vorgenommen werden.

Dazu kann in der Steuereinrichtung 13 ein Regelprozess implementiert sein, welcher die Laserleistung anhand der mittels einer Temperatur-Messeinrichtung gemessenen Temperatur regelt, um eine vorgegebene Temperatur oder ein vorgegebenes Temperatur/Zeit-Profil am Glas-Vorprodukt einzustellen. Als Temperatur-Messeinrichtung ist bei dem in Fig. 1 gezeigten Beispiel ein Pyrometer 11 vorgesehen, welches die Wärmestrahlung des Glasrohres an dessen durch den Laser 5 erwärmten Ende 31 misst. Die Messwerte werden der Steuereinrichtung 13 zugeführt und im Regelprozess zur Einstellung der gewünschten Temperatur verwendet.

Besonders vorteilhaft bei einer Anordnung, wie sie beispielhaft Fig. 1 zeigt, ist, dass das Laserlicht die Formwerkzeuge nicht direkt erwärmt. Dies führt dazu, dass die Formwerkzeuge trotz einer Erwärmung des Glas-Vorprodukts während der Umformung im Allgemeinen nicht stärker erwärmt werden als bei einem Prozess mit vorangehender Erwärmung durch Brenner. Insgesamt wird auf diese Weise weniger Wärmeenergie erzeugt und diese Wärmeenergie auch noch gezielter in das Glas-Vorprodukt eingebracht. Damit werden insgesamt die Aufheizung der gesamten Vorrichtung und damit unter anderem durch Wärmedehnungen entstehende Einlaufphänomene reduziert.

Ein bevorzugtes Glas für die Fertigung von Spritzenkörpern ist Borosilikatglas. Besonders bevorzugt wird dabei alkaliarmes Borosilikatglas, insbesondere mit einem Alkaligehalt von weniger als 10 Gewichtsprozenten. Borosilikatglas eignet sich generell gut aufgrund der typischerweise hohen Temperaturwechselbeständigkeit. Diese ist günstig, um bei den schnellen Prozesszeiten, wie sie mit der Erfindung erzielbar sind, schnelle Aufheizrampen realisieren zu können.

Ein geeignetes alkaliarmes Borosilikatglas weist folgende Bestandteile in Gewichtsprozent auf:

| | |
|---|---|
| SiO₂ | 75 Gew% |
| B₂O₃ | 10,5 Gew % |
| Al₂O₃ | 5 Gew % |
| Na₂O | 7 Gew % |
| CaO | 1,5 Gew % |

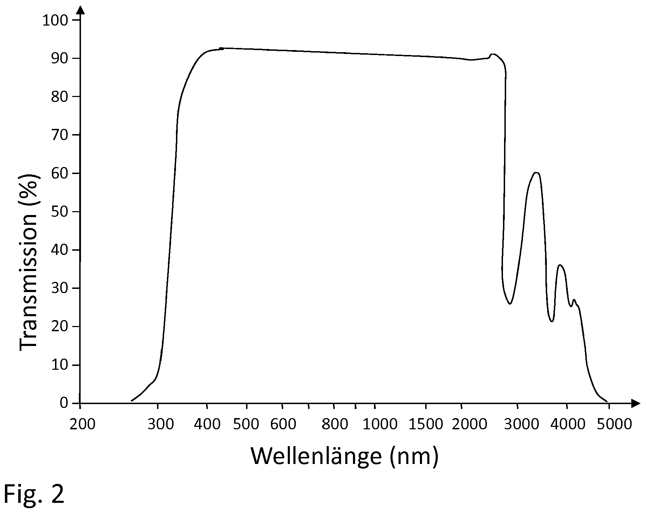

Ein Transmissionsspektrum des Glases zeigt Fig. 2. Die angegebenen Transmissionswerte beziehen sich auf eine Glasdicke von einem Millimeter. Anhand von Fig. 2 ist ersichtlich, dass die Transmission des Glases bei Wellenlängen oberhalb von 2,5 Mikrometern absinkt. Oberhalb von 5 Mikrometern ist das Glas auch bei sehr dünnen Glasdicken praktisch opak.

Die in Fig. 2 gezeigte Abnahme der Transmission im Wellenlängenbereich oberhalb von 2,5 Mikrometern ist nicht wesentlich von der genauen Zusammensetzung des Glases abhängig. So können bei ähnlichen Transmissionseigenschaften die oben angegebenen Gehalte der Bestandteile bevorzugter Borosilikatgläser können auch um jeweils 25 % vom angegebenen Wert variieren. Weiterhin können außer Borosilikatglas selbstverständlich auch andere Gläser eingesetzt werden. Sofern diese bei der Wellenlänge des Lasers höchstens teiltransparent sind, kann zur Erhitzung eine Laserquelle verwendet werden.

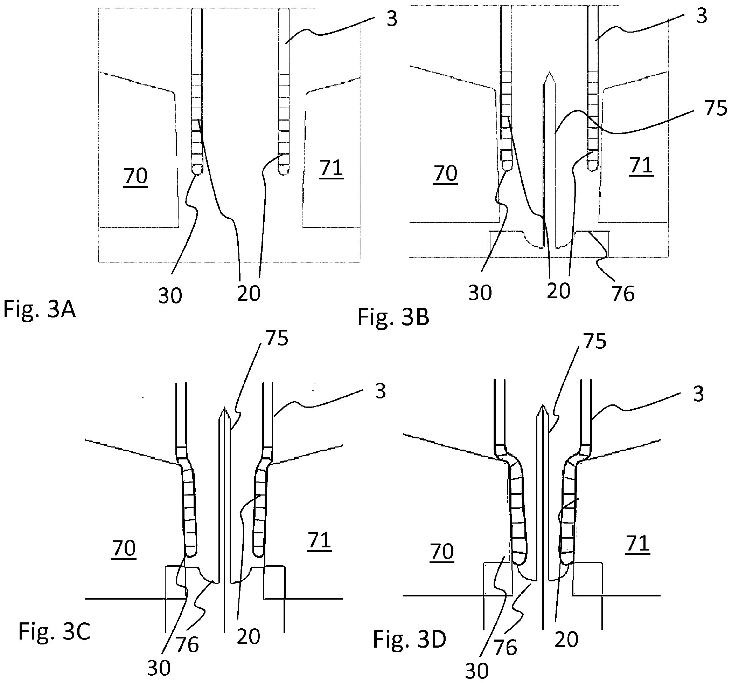

Die Figuren 3A bis 3F zeigen anhand von Schnittansichten schematisch eine Simulation eines erfindungsgemäßen Umformprozesses zur Ausformung eines Spritzenkonus aus einem Rohrglas 3 für die Herstellung eines Spritzenkörpers. Die Schnitte der Darstellungen verlaufen entlang der Mittenachse des Rohrglases 3, um welche das Rohrglas rotiert wird. Ebenfalls zu erkennen sind die Walzen 70, 71 und der Formdorn 75.

Die in den Schnittansichten des Rohrglases eingezeichneten, anfänglich senkrecht zur Mittenachse des Rohrglases verlaufenden Linien 20 kennzeichnen gedachte Grenzlinien von axialen Abschnitten des Rohrglases 3. Anhand dieser Linien wird der Materialfluss bei der Umformung kenntlich gemacht.

Der Formdorn 75 ragt aus einem Fuß 76 heraus, der zur Formung der vorderen Konusfläche der Spritze dient. Der Fuß 76 ist ein senkrecht zur Betrachtungsrichtung der Fig. 3A bis 3F flach ausgebildetes Bauteil. Anders als dargestellt ist in der tatsächlichen Vorrichtung der Fuß dabei um 90° um die Längsachse des Formdorns 75 gedreht, so dass der Fuß 76 zwischen die Walzen 70, 71 passt. Die Überlappung von Walzen 70, 71 und Fuß 76, wie sie ab Fig. 3C zu erkennen ist, tritt also tatsächlich nicht auf.

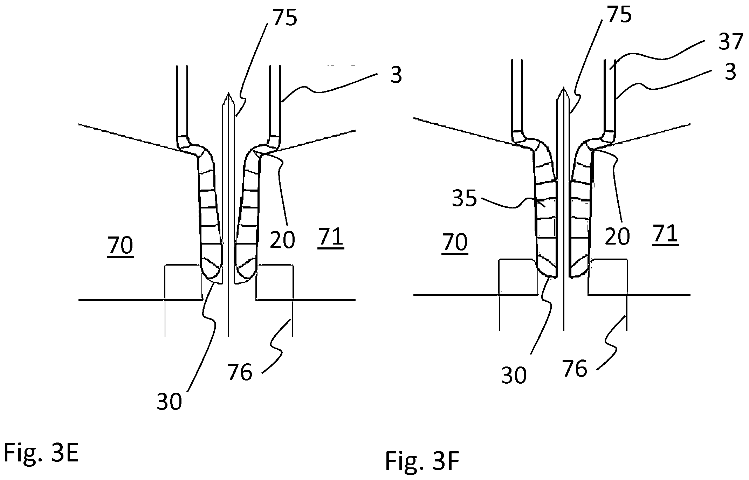

Eine Berührung der Walzen 70, 71 und die beginnende Verformung erfolgt ab der in Fig. 3C gezeigten Position. Es erfolgt nun eine Kompression des Rohrglases 3 durch die radial einwärts zur Mittenachse des Rohrglases bewegten Walzen 70, 71. Bei dem in Fig. 3E gezeigten Stadium berührt der Formdorn 75 innenseitig das Rohrglas und formt den Kanal des Spritzenkonus. Bei dem in Fig. 3F gezeigten Stadium schließlich ist die Ausformung des Spritzenkonus bereits beendet. Anschließend werden die Formwerkzeuge vom ausgeformten Spritzenkonus 35 weggefahren. Alle Formungsschritte zur Ausformung des Spritzenkonus 35 wurden mithin mit den gleichen Formwerkzeugen 70, 71, 75 und dem Fuß 76 durchgeführt. Eine derartige Umformungsstation führt daher alle Heiß-Umformungsschritte an einem Abschnitt des Glas-Vorprodukts aus. Es kann sich nun eine Ausformung des Spritzenflansches, beziehungsweise der Fingerauflage am anderen Ende des Rohrglases erfolgen.

Ab dem Verformungsstadium, wie es in Fig. 3E dargestellt ist, ist gut zu erkennen, dass die radiale Kompression am Spritzenkonus 35 zu einer Verdickung der Wandstärke führt. Hier besteht nun die Möglichkeit, durch die Einstellung einer entsprechenden Temperaturverteilung wie oben beschrieben, einen gewissen Materialfluss vom Ende 30 weg zu erzeugen. Ebenso kann es an den umlaufenden Kanten des ausgeformten Rohrglases im Übergangsbereich zwischen Spritzenzylinder 37 und Spritzenkonus 35 zu einer verminderten Wandstärke kommen. Auch diesem Effekt kann durch Einstellung eines axial inhomogenen Leistungseintrags über die Regelung der axialen Verteilung der Laserleistung begegnet werden.

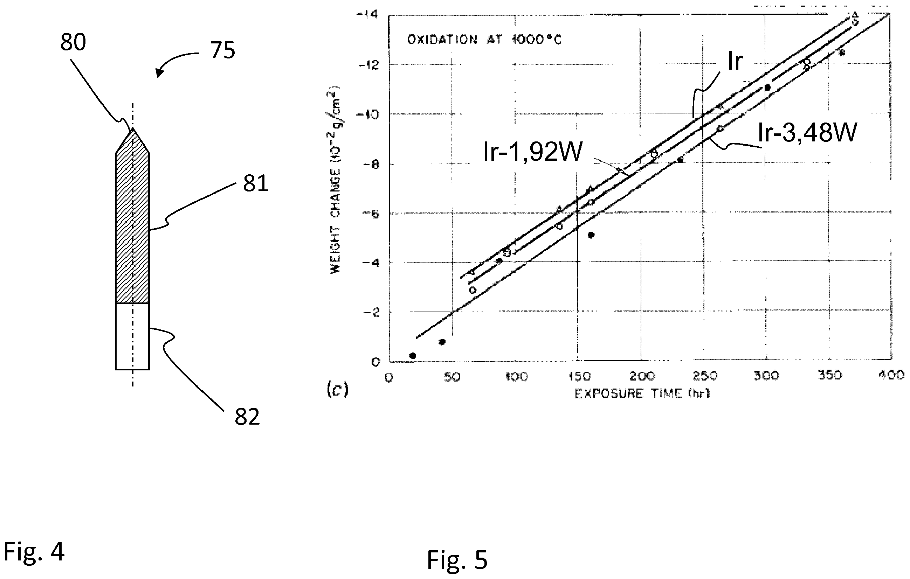

In Fig. 4 ist ein Abschnitt eines Formdorns 75 in einer Draufsicht dargestellt. Ausgehend von der Spitze 80 des Formdorns 75 ist ein an die Spitze angrenzender Bereich 81 des Formdorns 75 markiert, in dem der Formdorn 75 während des Umformprozesses in Kontakt mit dem Glas-Vorprodukt kommt oder kommen kann. Ein Bereich 82 ist vorgesehen zum Halten des Formdorns 75.

Der Formdorn 75 ist erfindungsgemäß aus einer Legierung gebildet, welche zumindest ein erstes temperaturstabiles Kernmaterial aufweist. Das Kernmaterial ist also das Grund- oder Basismaterial des Formdorns 75 und ist dabei ausgewählt aus der Gruppe der Edelmetalle, insbesondere der Platingruppenelemente. Demnach handelt es sich um einen massiven Formdorn, zumindest in demjenigen Bereich 81, der dem Umformen dient. Vorzugsweise ist der Formdorn 75 monolithisch ausgebildet.

Im vorliegenden Beispiel ist das Kernmaterial ein Edelmetall, vorzugsweise Platin oder Iridium, oder eine entsprechende Platin- oder Iridium-Legierung. Das Kernmaterial des Formdorns 75 kann neben Iridium und/oder Platin, Palladium, Rhodium, Rhenium und/oder Ruthenium oder eine entsprechende Legierung aufweisen.

Neben dem Kernmaterial weist der Formdorn 75 ein weiteres Legierungselement auf. Dieses ist ausgewählt aus der Gruppe umfassend Wolfram, Zirkonium, Rhodium, Molybdän oder Rhenium. Der Umfang dieser Legierungselemente liegt dabei vorteilhaft in einem Bereich von wenigstens 0,1 Gew.-%, bevorzugt von wenigstens 0,3 Gew.-%, besonders bevorzugt 0,5 Gew.-% und ganz besonders bevorzugt wenigstens 0,9 Gew.-% sowie höchstens 10 Gew.-%, bevorzugt höchstens 6 Gew.-% und besonders bevorzugt höchstens 5 Gew.-%.

In einer ersten Ausführungsform umfasst das Kernmaterial des Formdorns 75 wenigstens 20 Gew.-% Platin oder eine Legierung mit wenigstens 20 Gew.-% Platin.

In einer weiteren Ausführungsform umfasst das Kernmaterial wenigstens 5 Gew.-% Rhodium oder eine Legierung mit wenigstens 5 Gew.-% Rhodium.

In einer weiteren, bevorzugten Ausführungsform umfasst das Kernmaterial eine Platin-Rhodium-Legierung mit wenigstens 20 Gew.-% Platin und wenigstens 5 Gew.-% Rhodium.

In einer nochmaligen weiteren Ausführungsform umfasst das Kernmaterial eine Platin-Nickel-Legierung mit wenigstens 5 Gew.-% Nickel.

In einer nochmaligen weiteren, bevorzugten Ausführungsform umfasst das Kernmaterial eine Platin-Iridium-Legierung mit wenigstens 5 Gew.-%, bevorzugt wenigstens 10 Gew.-% und besonders bevorzugt wenigstens 20 Gew.-% Iridium.

In einer nochmaligen weiteren, bevorzugten Ausführungsform umfasst das Kernmaterial wenigstens 20 Gew.-%, bevorzugt wenigstens 50 Gew.-%, besonders bevorzugt wenigstens 70 Gew.-% und ganz besonders bevorzugt wenigstens 90 Gew.-% Iridium als Legierungselement.

In einer ganz besonders bevorzugten Ausführungsform ist als Kernmaterial für den Formdorn 75 Iridium ausgewählt und als Legierungselement Wolfram.

Sehr geeignet für das Umformen eines Rohrglases aus Borosilikatglas mit einem Formwerkzeug 1 wie vorstehend beschrieben ist ein Material für den Formdorn 75, welches eine Iridium-Wolfram-Legierung mit wenigstens 0,01 Gew.-%, 0,05 Gew.-%, 0,1 Gew.-%, bevorzugt wenigstens 0,3 Gew.-%, besonders bevorzugt wenigstens 0,5 Gew.-% und ganz besonders bevorzugt wenigstens 1 Gew.-% Wolfram umfasst. Dabei ist es weiterhin vorteilhaft, wenn der Wolfram-Anteil höchstens 10 Gew.-%, bevorzugt höchstens 6 Gew.-% und besonders bevorzugt höchstens 5 Gew.-% beträgt. Ein besonders geeigneter Formdorn 75 umfasst eine Iridium-Wolfram-Legierung mit 0,96 Gew.-%, 1,92 Gew.-% oder 3,84 Gew.-% Wolfram.

Die vergleichsweise hohe Härte und die hohe Rekristallisiationstemperatur von Iridum wirken sich sehr positiv auf die Standzeit des Formdorns 75 beim Umformen von Borosilikatglas aus. Dies ermöglicht auch Umformvorgänge bei Temperaturen über 800°C oder über 900°C. Begünstigt wird dies weiterhin durch die hohe Säurebeständigkeit von Wolfram.

Der Formdorn 75 zeigte auch nach sehr vielen Bearbeitungszyklen oder Umformprozessen, etwa zwischen 8.000 und 10.000 Zyklen, keine Tendenz zur Rissbildung auf der Oberfläche oder andere Abnutzungserscheinungen.

Aufgrund der geringen Verschleißerscheinungen, insbesondere aufgrund des geringen Materialabtrags des Formdorns 75 mit einer Iridium-Wolfram-Legierung, konnte ferner beobachtet werden, dass der Grad der Verunreinigung mit Wolfram sehr niedrig war.

Es konnte beobachtet werden, dass die Verwendung eines Formdorns 75 auf Basis einer Iridium-Wolfram-Legierung mit einem Wolfram-Anteil von etwa 1 Gew.-% zu einer besonders hohen Standzeit des Formdorns 75 führt. Dieser geringe Materialabtrag hat zugleich den Vorteil, dass die Kontamination mit Wolfram besonders gering ist.

Spritzen aus Glas, vorzugsweise aus Borosilikatglas, welche umgeformt wurden mittels des vorstehend genannten Formwerkzeuges, wiesen innerhalb des umgeformten Hohlraumes einen sehr niedrigen Wolframgehalt auf.

Es können Spritzen mit dem Formdorn 75 umgeformt werden, deren Wolframgehalt innerhalb des Hohlraumes der Spritze bei weniger als 50 ng, vorzugsweise bei weniger als 30 ng, noch bevorzugter bei weniger als 10 ng pro Spritze liegt.

In einer besonders bevorzugten Ausführungsform beträgt der Wolframgehalt innerhalb des Hohlraumes einer mit dem Formdorns 75 umgeformten Spritze zwischen 1 und 10 ng pro Spritze, vorzugsweise zwischen 1 und 5 ng pro Spritze und besonders bevorzugt zwischen 1 und 3 ng.

Die Erzeugung derartiger Spritzen konnte so mit deutlichen Kosteneinsparungen realisiert werden.

In Fig. 5 ist der eine Übersicht über den Massenverlust verschiedener Iridium-Wolfram-Legierungen bei 1.000 °C angegeben (Quelle: Liu, Inouye, Dez. 1976, Oak Ridge National Labaratory, Tennesee). In der Übersicht sind auf der Ordinatenachse Zeiten in Stunden und auf der Abszissenachse die Gewichtsveränderung in 10⁻² g/cm2.

Proben aus drei unterschiedlichen Legierungen wurden einer Temperatur von 1.000°C ausgesetzt und der Materialabtrag wurde gemessen. Neben reinem Iridium wurden zwei Iridium-Wolfram-Legierungen mit 1,92 Gew.-% Wolfram und 3,84 Gew.-% Wolfram (Ir-1.94W, Ir-3.84W) in ihrem Materialabtrag miteinander verglichen. Die Übersicht zeigt, dass der Materialverlust von dem Gehalt an Wolfram abhängt und bei höheren Wolframanteilen abnimmt.

Die Vorteile von Iridium-Wolfram-Legierungen liegen in zum einen in der hohen Standzeit, verbunden mit einer hohen Dimensionstoleranz. So ist der Massenverlust durch Bildung flüchtiger Oxide verglichen mit reinem Ididium durch den Wolfram Gehalt deutlich reduziert, wodurch kein messbarer Materialabtrag am Iridiumdorn zu beobachten ist. Durch den geringen Abtrag am Formdorn ist eine gleichbleibend hohe Dimensionstoleranz des Konuskanals während der Fertigung möglich mit einem engen Toleranzfeld möglich.

Ferner zeichnet sich der auf einer Iridium-Wolfram-Legierung basierende Formdorn durch eine geringe Reaktivität gegenüber den Glasbestandteilen aus. So besitzt Iridium als Edelmetall gegenüber den Glasverdampungsprodukten, etwa Alkalioxiden oder Boroxiden, nur eine untergerodnete Reaktivität, wodurch die Korrosion weitgehend unterdrückt ist.

Der Verureinigungseintrag in das umgeformnte Glas-Produkt ist sehr gering und chemisch analytisch kaum messbar.

Zudem werden keine Ablagerungen, etwa Oxidpartikel, im Konuskanal gebildet.

Das erfindungsgemäße Formwerkzeug, insbesondere der erfindungsgemäße Formdorn 75, bringen demnach vielfältige Vorteile mit sich, die das Formwerkzeug besonders geeignet erscheinen lassen für die Erzeugung von wolframarmen Pharmapackmitteln, wie insbesondere Spritzen.

## Patentansprüche

1. Formwerkzeug (7) zum Umformen von
hohlkörperförmigen Glas-Vorprodukten (3), umfassend einen Formdorn (75) zum Umformen wenigstens eines Abschnittes eines erhitzten Bereiches des Glas-Vorproduktes (3), wobei
der Formdorn (75) aus einer Legierung gebildet ist, welche zumindest ein erstes temperaturstabiles Kernmaterial als Basismaterial des Formdorns (75) und zumindest ein weiteres Legierungselement aufweist, wobei
das Kernmaterial ausgewählt ist aus der Gruppe, die Iridium, Palladium, Platin, Rhodium, Rhenium und/oder Ruthenium oder eine hierauf basierende Legierung umfasst, wobei
das zumindest eine weitere Legierungselement ausgewählt ist aus der Gruppe umfassend Wolfram, Zirkonium, Rhodium, Molybdän, Ruthenium oder Rhenium, und wobei Wolfram mit einem Anteil von wenigstens 0,01 Gew.-% enthalten ist.

2. Formwerkzeug (7) nach dem vorstehenden Anspruch, **dadurch gekennzeichnet, dass** das Kernmaterial wenigstens 20 Gew.-% Platin umfasst.

3. Formwerkzeug (7) nach einem der vorstehenden Ansprüche, **dadurch gekennzeichnet, dass** das Kernmaterial wenigstens 5 Gew.-% Rhodium umfasst.

4. Formwerkzeug (7) nach einem der vorstehenden Ansprüche, **dadurch gekennzeichnet, dass** das Kernmaterial eine Platin-Rhodium-Legierung mit wenigstens 20 Gew.-% Platin, bevorzugt wenigstens 25 Gew.-% Platin und besonders bevorzugt wenigstens 30 Gew.-% Platin und wenigstens 5 Gew.-% Rhodium, bevorzugt wenigstens 7 Gew.-% Rhodium und besonders bevorzugt wenigstens 10 Gew.-% Rhodium umfasst.

5. Formwerkzeug (7) nach einem der vorstehenden Ansprüche, **dadurch gekennzeichnet, dass** das Kernmaterial eine Platin-Nickel-Legierung mit wenigstens 5 Gew.-% Nickel umfasst.

6. Formwerkzeug (7) nach einem der vorstehenden Ansprüche, **dadurch gekennzeichnet, dass** das Kernmaterial eine Platin-Iridium-Legierung mit wenigstens 5 Gew.-% Iridium, bevorzugt wenigstens 10 Gew.-% Iridium und besonders bevorzugt wenigstens 20 Gew.-% Iridium umfasst.

7. Formwerkzeug (7) nach einem der vorstehenden Ansprüche, **dadurch gekennzeichnet, dass** das Kernmaterial wenigstens 20 Gew.-% Iridium, bevorzugt wenigstens 50 Gew.-% Iridium, besonders bevorzugt wenigstens 70 Gew.-% Iridium und ganz besonders bevorzugt wenigstens 90 Gew.-% Iridium umfasst.

8. Formwerkzeug (7) nach einem der vorstehenden Ansprüche, **dadurch gekennzeichnet, dass** das weitere Legierungselement Wolfram mit einem Anteil von wenigstens 0,05 Gew.-%, 0,1 Gew.-%, bevorzugt von wenigstens 0,3 Gew.-%, besonders bevorzugt 0,5 Gew.-% und ganz besonders bevorzugt wenigstens 0,9 Gew.-% umfasst.

9. Formwerkzeug (7) nach einem der vorstehenden Ansprüche, **dadurch gekennzeichnet, dass** das weitere Legierungselement Wolfram mit einem Anteil von höchstens 10 Gew.-%, bevorzugt höchstens 6 Gew.-% und besonders bevorzugt höchstens 5 Gew.-% umfasst.

10. Formwerkzeug (7) nach einem der vorstehenden Ansprüche, **dadurch gekennzeichnet, dass** das Formwerkzeug (7) eine Iridium-Wolfram-Legierung mit wenigstens 0,1 Gew.-%, bevorzugt wenigstens 0,3 Gew.-% und besonders bevorzugt wenigstens 0,5 Gew.-% Wolfram umfasst.

11. Formwerkzeug (7) nach dem vorstehenden Anspruch, **dadurch gekennzeichnet, dass** die Iridium-Wolfram-Legierung 0,96 Gew.-% Wolfram, 1,92 Gew.-% Wolfram oder 3,84 Gew.-% Wolfram umfasst.

12. Vorrichtung (1) zum Umformen von hohlkörperförmigen Glas-Vorprodukten (3), umfassend
- eine Einrichtung zur lokalen Erhitzung eines Bereichs eines Glas-Vorprodukts (3) bis über dessen Erweichungspunkt, und
- zumindest ein Formwerkzeug (7) zum Umformen wenigstens eines Abschnittes eines mit der Einrichtung zur lokalen Erhitzung erhitzten Bereichs eines Glas-Vorprodukts (3),
- wobei das Formwerkzeug (7) einen Formdorn (75) gemäß einem der vorstehenden Ansprüche umfasst.

13. Vorrichtung (1) zum Umformen von hohlkörperförmigen Glas-Vorprodukten (3) nach vorstehendem Anspruch, wobei die Einrichtung zur lokalen Erhitzung einen Laser (5) umfasst,
- wobei eine Rotationseinrichtung (9) vorgesehen ist, um das Formwerkzeug (7) und das Glas-Vorprodukt (3) relativ zueinander zu rotieren, und wobei
- das Formwerkzeug (7) so ausgebildet ist, dass ein Oberflächenbereich des umzuformenden Abschnittes des Glas-Vorproduktes (3) nicht durch das Formwerkzeug (7) abgedeckt wird, wobei der Laser (5) oder eine dem Laser (5) nachgeschaltete Optik so angeordnet ist, dass das Laserlicht bei der Umformung auf den nicht vom Formwerkzeug (7) abgedeckten Bereich eingestrahlt wird, und wobei eine Steuereinrichtung (13) vorgesehen ist, welche den Laser (5) so ansteuert, dass ein Glas-Vorprodukt (3) zumindest zeitweise während der Umformung durch das Laserlicht erwärmt wird.

14. Verfahren zur Herstellung eines Pharmapackmittels aus Glas, bei welchem
- lokal ein hohlkörperförmiges Glas-Vorprodukt (3) bis über dessen Erweichungspunkt erhitzt, und
- mit einem Formwerkzeug (7) nach einem der vorstehenden Ansprüche 1 bis 11 wenigstens ein Abschnitt eines mit einer Einrichtung zur lokalen Erhitzung erhitzten Bereiches des Glas-Vorproduktes (3) umgeformt wird,
- wobei das Formwerkzeug (7) einen Formdorn (75) nach einem der vorstehenden Ansprüche 1 bis 11 umfasst.

15. Spritze aus Glas, hergestellt oder herstellbar mit einem Formwerkzeug (7) nach einem der vorstehenden Ansprüche 1 bis 11, **dadurch gekennzeichnet, dass** sie einen sehr geringen Gehalt an Wolfram innerhalb des Hohlraums einschließlich des inneren, umgeformten Konusbereiches, aufweist und der W-Gehalt höchstens 100 ng, höchstens 50 ng, höchstens 10 ng, bevorzugt höchstens 1 ng und besonders bevorzugt höchstens 0,1 ng pro Spritze beträgt.

16. Spritze aus Glas nach vorstehendem Anspruch, **dadurch gekennzeichnet, dass** der Gehalt an Wolfram pro Spritze bei > 0 ng, > 0,006 ng, > 0,01 ng, > 0,03 ng liegt.

## Claims

1. A moulding tool (7) for reshaping hollow-body glass precursors (3), comprising:
a forming mandrel (75) for reshaping at least a portion of a heated zone of the glass precursor (3); wherein
the forming mandrel (75) is made of an alloy comprising at least a first temperature-stable core material as the basic material of the forming mandrel (75) and at least one further alloying element; wherein
the core material is selected from the group comprising iridium, palladium, platinum, rhodium, rhenium, and/or ruthenium, or an alloy based thereon; wherein
the at least one further alloying element is selected from the group comprising tungsten, zirconium, rhodium, molybdenum, ruthenium, and rhenium; and
wherein tungsten is included with an amount of at least 0.01 wt%.

2. The moulding tool (7) according to the preceding claim, **characterised in that** the core material comprises at least 20 wt% of platinum,

3. The moulding tool (7) according to any one of the preceding claims, **characterised in that** the core material comprises at least 5 wt% of rhodium.

4. The moulding tool (7) according to any one of the preceding claims, **characterised in that** the core material is a platinum-rhodium alloy including at least 20 wt% of platinum, preferably at least 25 wt% of platinum, and more preferably at least 30 wt% of platinum, and at least 5 wt% of rhodium, preferably at least 7 wt% of rhodium, and more preferably at least 10 wt% of rhodium.

5. The moulding tool (7) according to any one of the preceding claims, **characterised in that** the core material is a platinum-nickel alloy including at least 5 wt% of nickel.

6. The moulding tool (7) according to any one of the preceding claims, **characterised in that** the core material is a platinum-indium alloy including at least 5 wt% of iridium, preferably at least 10 wt% of iridium, and more preferably at least 20 wt% of iridium.

7. The moulding tool (7) according to any one of the preceding claims, **characterised in that** the core material comprises at least 20 wt% of iridium, preferably at least 50 wt% of iridium, more preferably at least 70 wt% of iridium, and most preferably at least 90 wt% of iridium.

8. The moulding tool (7) according to any one of the preceding claims, **characterised in that** the further alloying element is tungsten with an amount of at least 0.05 wt%, 0.1 wt%, preferably at least 0.3 wt%, more preferably 0.5 wt%, and most preferably at least 0.9 wt%.

9. The moulding tool (7) according to any one of the preceding claims, **characterised in that** the further alloying element is tungsten with an amount of not more than 10 wt%, preferably not more than 6 wt%, and most preferably not more than 5 wt%.

10. The moulding tool (7) according to any one of the preceding claims, **characterised in that** the moulding tool (7) comprises an iridium-tungsten alloy with at least 0.1 wt%, preferably at least 0.3 wt%, and most preferably at least 0.5 wt% of tungsten.

11. The moulding tool (7) according to any one of the preceding claims, **characterised in that** the iridium-tungsten alloy comprises 0.96 wt% of tungsten, 1.92 wt% of tungsten, or 3.84 wt% of tungsten.

12. An apparatus (1) for reshaping hollow-body glass precursors (3), comprising:
- means for locally heating a zone of a glass precursor (3) to above the softening point thereof; and
- at least one moulding tool (7) for reshaping at least a portion of the zone of a glass precursor (3) heated by said means for locally heating;
- wherein the moulding tool (7) comprises a forming mandrel (75) according to any one of the preceding claims.

13. The apparatus for reshaping hollow-body glass precursors (3) according to the preceding claim, wherein said means for locally heating comprise a laser (5);
- wherein rotating means (9) are provided for rotating the moulding tool (7) and the glass precursor (3) relative to each other; and wherein
- the moulding tool (7) is configured so that a surface area of the portion of the glass precursor (3) to be reshaped is not covered by the moulding tool (7);
wherein the laser (5) or an optic system downstream of the laser (5) is arranged so that during reshaping the laser light is irradiated onto the area not covered by the moulding tool (7); and wherein
a control device (13) is provided which controls the laser (5) so that a glass precursor (3) is at least temporarily heated by the laser light during the reshaping.

14. A method for producing a pharmaceutical packaging made of glass, wherein:
- a hollow-body glass precursor (3) is locally heated to above its softening point; and
- at least a portion of a zone of the glass precursor (3) heated by means for locally heating is reshaped using a moulding tool (7) according to any one of the preceding claims 1 to 11;
- wherein the moulding tool (7) comprises a forming mandrel (75) according to any one of the preceding claims 1 to 11.

15. A glass syringe, produced or producible using a moulding tool (7) according to any one of the preceding claims 1 to 11, **characterised by** having a very low content of tungsten in particular in the cavity including the reshaped inner cone portion, and wherein the tungsten content is not more than 100 ng, not more than 50 ng, preferably not more than 10 ng, more preferably not more than 1 ng, and most preferably not more than 0.1 ng per syringe.

16. The glass syringe according to the preceding claim, **characterised in that** the tungsten content per syringe is > 0 ng, > 0.006 ng, > 0.01 ng, > 0.03 ng.

## Revendications

1. Outil de formage (7) destiné à la mise en forme de produits semi-finis en verre (3) en forme de corps creux, comprenant un mandrin de formage (75) destiné à la mise en forme d'au moins une partie d'une zone chauffée du produit semi-fini en verre (3), sachant que
le mandrin de formage (75) est constitué d'un alliage qui présente au moins un premier matériau de noyau à stabilité en température, en tant que matériau de base du mandrin de formage (75), et au moins un élément d'alliage supplémentaire, sachant que
le matériau de noyau est choisi dans le groupe comprenant l'iridium, le palladium, le platine, le rhodium, le rhénium et/ou le ruthénium ou un alliage basé sur ceux-ci, sachant que
l'élément d'alliage supplémentaire, au nombre d'au moins un, est choisi dans le groupe comprenant le tungstène, le zirconium, le rhodium, le molybdène, le ruthénium ou le rhénium, et sachant que le tungstène est présent en une proportion d'au moins 0,01 % en poids.

2. Outil de formage (7) selon la revendication précédente, **caractérisé en ce que** le matériau de noyau comporte au moins 20 % en poids de platine.

3. Outil de formage (7) selon l'une des revendications précédentes, **caractérisé en ce que** le matériau de noyau comporte au moins 5 % en poids de rhodium.

4. Outil de formage (7) selon l'une des revendications précédentes, **caractérisé en ce que** le matériau de noyau comporte un alliage platine-rhodium avec au moins 20 % en poids de platine, de préférence au moins 25 % en poids de platine, et de façon particulièrement avantageuse au moins 30 % en poids de platine, et avec au moins 5 % en poids de rhodium, de préférence au moins 7 % en poids de rhodium, et de façon particulièrement avantageuse au moins 10 % en poids de rhodium.

5. Outil de formage (7) selon l'une des revendications précédentes, **caractérisé en ce que** le matériau de noyau comporte un alliage platine-nickel avec au moins 5 % en poids de nickel.

6. Outil de formage (7) selon l'une des revendications précédentes, **caractérisé en ce que** le matériau de noyau comporte un alliage platine-iridium avec au moins 5 % en poids d'iridium, de préférence au moins 10 % en poids d'iridium, et de façon particulièrement avantageuse au moins 20 % en poids d'iridium.

7. Outil de formage (7) selon l'une des revendications précédentes, **caractérisé en ce que** le matériau de noyau comporte au moins 20 % en poids d'iridium, de préférence au moins 50 % en poids d'iridium, de préférence notamment 70 % en poids d'iridium, et de façon particulièrement avantageuse au moins 90 % en poids d'iridium.

8. Outil de formage (7) selon l'une des revendications précédentes, **caractérisé en ce que** l'élément d'alliage supplémentaire comprend du tungstène en une proportion d'au moins 0,05 % en poids, 0,1 % en poids, de préférence d'au moins 0,3 % en poids, de préférence notamment 0,5 % en poids, et de façon particulièrement avantageuse d'au moins 0,9 % en poids.

9. Outil de formage (7) selon l'une des revendications précédentes, **caractérisé en ce que** l'élément d'alliage supplémentaire comprend du tungstène en une proportion d'au maximum 10 % en poids, de préférence d'au maximum 6 % en poids, et de façon particulièrement avantageuse d'au maximum 5 % en poids.

10. Outil de formage (7) selon l'une des revendications précédentes, **caractérisé en ce que** l'outil de formage (7) comporte un alliage iridium-tungstène avec au moins 0,1 % en poids, de préférence au moins 0,3 % en poids, et de façon particulièrement avantageuse au moins 0,5 % en poids de tungstène.

11. Outil de formage (7) selon la revendication précédente, **caractérisé en ce que** l'alliage iridium-tungstène comporte 0,96 % en poids de tungstène, 1,92 % en poids de tungstène ou 3,84 % en poids de tungstène.

12. Dispositif (1) destiné à la mise en forme de produits semi-finis en verre (3) en forme de corps creux, comprenant
- un dispositif de chauffage local d'une zone d'un produit semi-fini en verre (3), jusqu'au-delà du point de ramollissement de celui-ci, et
- au moins un outil de formage (7) destiné à la mise en forme d'au moins une partie d'une zone un produit semi-fini en verre (3) chauffée avec le dispositif de chauffage local,
- sachant que l'outil de formage (7) comprend un mandrin de formage (75) selon l'une des revendications précédentes.

13. Dispositif (1) destiné à la mise en forme de produits semi-finis en verre (3) en forme de corps creux selon la revendication précédente, le dispositif de chauffage local comprenant un laser (5),
- dans lequel il est prévu un dispositif de rotation (9) aux fins de mettre l'outil de formage (7) et le produit semi-fini en verre (3) en rotation l'un par rapport à l'autre, et dans lequel
- l'outil de formage (75) est agencé de manière à ce qu'une zone de surface de la partie du produit semi-fini en verre (3) devant être mise en forme ne soit pas recouverte par l'outil de formage (7), sachant que le laser (5) ou un système optique placé en aval du laser (5) est disposé de manière à ce que, lors du formage, la lumière laser soit envoyée sur la zone qui n'est pas recouverte par l'outil de formage (7), et sachant qu'il est prévu un dispositif de commande (13) qui active le laser (5) de manière à ce qu'un produit semi-fini en verre (3) soit chauffé par la lumière laser au moins temporairement pendant le formage.

14. Procédé de fabrication d'un produit d'emballage pharmaceutique en verre, selon lequel
- on chauffe localement un produit semi-fini en verre (3) en forme de corps creux, jusqu'au-delà du point de ramollissement de celui-ci, et
- on met en forme, à l'aide d'un outil de formage (7) selon l'une des revendications 1 à 11, au moins une partie d'une zone du produit semi-fini en verre (3) qui a été chauffée avec un dispositif de chauffage local,
- l'outil de formage (7) comprenant un mandrin de formage (75) se-Ion l'une des revendications précédentes 1 à 11.

15. Seringue en verre, fabriquée ou pouvant être fabriquée à l'aide d'un outil de formage (7) selon l'une des revendications précédentes 1 à 11, **caractérisée en ce qu'**elle présente une très faible teneur en tungstène à l'intérieur de la cavité, y compris de la zone conique intérieure mise en forme, et que la teneur en W est au maximum de 100 ng, au maximum de 50 ng, au maximum de 10 ng, de préférence au maximum de 1 ng, et de façon particulièrement avantageuse au maximum de 0,1 ng par seringue.

16. Seringue en verre selon la revendication précédente, **caractérisée en ce que** la teneur en tungstène par seringue est > 0 ng, > 0,006 ng, > 0,01 ng, > 0,03 ng.
